# EUROPÄISCHE PATENTSCHRIFT

(11) **EP 3 280 311 B1**
(45) Veröffentlichungstag und Bekanntmachung des Hinweises auf die Patenterteilung: **06.11.2024**
(21) Anmeldenummer: 16712004.7
(22) Anmeldetag: 18.03.2016
(51) Int. Cl.: A61B 1/00, A61B 1/12

(54) **ÜBERWACHEN DER VERBINDUNG VON FLUIDLEITUNGEN MIT CHIRURGISCHEN INSTRUMENTEN**
MONITORING OF THE CONNECTION OF FLUID LINES TO SURGICAL INSTRUMENTS
SURVEILLANCE DU RACCORD DE CONDUITES À FLUIDE AVEC DES INSTRUMENTS CHIRURGICAUX

(30) Priorität: 08.04.2015 DE 102015206267
(43) Veröffentlichungstag der Anmeldung: 14.02.2018
(73) Patentinhaber: Olympus Winter & Ibe GmbH, 22045 Hamburg (DE)
(72) Erfinder: BOETTCHER-WILMES, Marius, 23858 Reinfeld (DE)
(74) Vertreter: Seemann & Partner Patentanwälte mbB
(86) Internationale Anmeldenummer: PCT/EP2016/055983
(87) Internationale Veröffentlichungsnummer: WO 2016/162185

(56) Entgegenhaltungen:
- EP-A1- 2 642 609
- EP-A2- 2 080 486
- WO-A1-03/067138

## Beschreibung

Die Erfindung betrifft ein Überwachungssystem für die Aufbereitung von chirurgischen Instrumenten, wobei zur Zufuhr eines Fluids zu dem chirurgischen Instrument eine Fluidleitung vorgesehen ist, die mit dem chirurgischen Instrument verbindbar oder verbunden ist, wobei die Fluidleitung zum Verbinden mit dem chirurgischen Instrument einen Fluidleitungsanschluss aufweist. Die Erfindung betrifft ferner ein Verfahren zum Verbinden eines chirurgischen Instruments mit wenigstens einer Fluidleitung, die beim Aufbereiten des chirurgischen Instruments Fluid zum chirurgischen Instrument führt oder vom chirurgischen Instrument wegführt, wobei die wenigstens eine Fluidleitung an wenigstens einem Anschluss des chirurgischen Instrumentes angeschlossen wird. Außerdem betrifft die Erfindung ein Verfahren zum Betreiben einer Aufbereitungsvorrichtung zum Aufbereiten eines chirurgischen Instruments.

Aus DE 10 2012 218 811 A1 ist eine Aufbereitungsvorrichtung und ein Verfahren zum Betreiben einer entsprechenden Aufbereitungsvorrichtung bzw. eines Aufbereitungssystems bekannt. Hierbei werden entsprechende Körbe, die in eine Aufbereitungsvorrichtung eingebracht werden können, mit chirurgischen Instrumenten bestückt, wobei Fluidleitungen mit Instrumentenanschlüssen des chirurgischen Instruments verbunden werden. Um eine Aufbereitung der chirurgischen Instrumente zu ermöglichen, werden insbesondere entsprechende Kanäle des chirurgischen Instruments mit Fluid gespült.

Es ist bei der Aufbereitung von chirurgischen Instrumenten, wie beispielsweise Endoskopen bekannt, eine Dichtigkeitsprüfung bei der Innenkanalspülung von chirurgischen Instrumenten vorzunehmen, so dass bei einem fehlerhaft angeschlossenen Instrumentenadapter bzw. einer fehlerhaft angeschlossenen Fluidleitung während der Aufbereitung des chirurgischen Instruments ein Programmabbruch geschieht. Um eine Dichtigkeitsprüfung durchzuführen, kann beispielsweise bei einem nicht korrekt angeschlossenen Adapter ein gewisser Überdruck aufgebaut werden und geschaut werden, wie der Druckabfall sich innerhalb einer Testzeit entwickelt. Bei einer Innenkanalspülung wird zur Erkennung eines nicht korrekt angeschlossenen Adapters bzw. einer nicht korrekt angeschlossenen Fluidleitung in der Regel Wasser in der Aufbereitungsvorrichtung durch das chirurgische Instrument geführt und durch entsprechende Messung von Wasserdrücken oder Wassermengen erkannt, ob ein Anschluss fehlerhaft ist. In diesem Fall muss dann das Spülwasser zunächst abgepumpt werden, bevor der Anschluss korrigiert werden kann. Dieser Vorgang ist relativ zeitaufwändig.

EP 2 080 486 A2 zeigt eine Endoskop-Wasch- und Desinfizierungsvorrichtung. Die Vorrichtung erkennt, ob Flüssigkeitsleitungen korrekt an die vorgesehenen Anschlüsse angeschlossen sind.

In WO 03/067138 A1 ist eine Verbindungsvorrichtung für den Transport von Fluiden gezeigt. Zudem umfasst die Verbindungsvorrichtung einen Signalkontakt um eine elektrische Verbindung mit einem Signalleiter hertzstellen.

EP 2 642 609 A1 offenbart ein Steckverbinderteil für ein medizinisches Gerät oder Instrument. Das Steckverbinderteil umfasst Fluidsteckelemente, die mit elektrischen Kontakteinrichtungen versehen sind.

Es ist Aufgabe der vorliegenden Erfindung, eine sehr schnelle und effiziente Möglichkeit vorzusehen, einen korrekten Anschluss von Fluidleitungen an ein chirurgisches Instrument zur Verfügung zu stellen.

Gelöst wird diese Aufgabe durch ein Überwachungssystem für die Aufbereitung von chirurgischen Instrumenten, wobei zur Zufuhr eines Fluids zu dem chirurgischen Instrument eine Fluidleitung vorgesehen ist, die mit dem chirurgischen Instrument verbindbar oder verbunden ist, wobei die Fluidleitung zum Verbinden der Fluidleitung mit dem chirurgischen Instrument einen Fluidleitungsanschluss aufweist, wobei der Fluidleitungsanschluss bei funktionsgemäßem Anschluss an das chirurgische Instrument einen elektrischen Kontakt schließt, das dadurch weitergebildet ist, dass der elektrische Kontakt dadurch ermöglicht wird, dass eine Außenfläche der Fluidleitung, die elektrisch leitend ist, mit einer Außenfläche des chirurgischen Instruments oder eines Instrumentenanschlusses des chirurgischen Instruments, der wenigstens abschnittsweise leitend ist, einen Kontakt schließt.

Unter einem funktionsgemäßen Anschluss wird im Rahmen der Erfindung ein fluiddichter Anschluss verstanden, durch den ein sicheres Spülen, und zwar leckfreies Spülen des chirurgischen Instruments, insbesondere von einem Lumen oder eines Kanals oder mehrerer Kanäle im chirurgischen Instrument, ermöglicht ist. Zudem wird unter einem funktionsgemäßen Anschluss ein Anschluss verstanden, bei dem eine definierte oder vorgebbare Leckage vorgesehen ist. Über diese Leckage können auch Außenflächen des Anschlusses mit Fluid, wie beispielsweise eine Desinfektionslösung, beaufschlagt werden. Hierdurch ist eine vollflächige Desinfektion möglich. Ein funktionsgemäßer Anschluss liegt insbesondere dann vor, wenn sichergestellt ist, dass auch bei den höchstmöglichen verwendeten Drücken bei der Spülung mittels eines Fluids der Anschluss der Fluidleitung an dem chirurgischen Instrument sicher angeschlossen bleibt.

Dieses wird erfindungsgemäß dadurch sichergestellt, dass erst bei einem funktionsgemäßen Anschluss des Fluidleitungsanschlusses an das chirurgische Instrument ein elektrischer Kontakt schließt, der dazu dienen kann, einer Bedienperson kenntlich zu machen, dass ein funktionsgemäßer Anschluss vorliegt.

Im Rahmen der Erfindung wird unter einem Schließen eines elektrischen Kontaktes verstanden, dass über den elektrischen Kontakt ein elektrischer Strom fließen kann. Der elektrische Kontakt wird ermöglicht dadurch, dass eine Außenfläche der Fluidleitung, die elektrisch leitend ist, mit einer Außenfläche des chirurgischen Instruments oder eines Instrumentenanschlusses des chirurgischen Instruments, der wenigstens abschnittsweise leitend ist, ein Kontakt geschlossen wird.

Vorzugsweise wird beim Schließen des elektrischen Kontakts ein von einer Bedienperson wahrnehmbares Signal generiert. Bei dem wahrnehmbaren Signal kann es sich um einen Ton oder eine optische Darstellung handeln, wie beispielsweise eine optische Darstellung auf einem Monitor oder einer Anzeigevorrichtung des Überwachungssystems. Es kann alternativ oder ergänzend hierzu eine Bewegung, wie beispielsweise ein Vibrieren, erzeugt werden.

Vorzugsweise ist eine elektrische Verbindung von dem elektrischen Kontakt, insbesondere über die Fluidleitung, zu einer Steuervorrichtung des Überwachungssystems vorgesehen. Hierdurch kann auf einfache Weise die Steuervorrichtung dazu dienen, Signale zu generieren, die von der Bedienperson wahrnehmbar sind.

Vorzugsweise ist die Fluidleitung ein Schlauch.

Wenn die elektrische Verbindung vorzugsweise eine in oder an der Fluidleitung angeordnete leitfähige Litze oder eine leitfähige Schicht ist oder umfasst, ist ein besonders bedienungsfreundliches Überwachungssystem vorgesehen. Die elektrisch leitfähige Litze oder die elektrisch leitfähige Schicht ist vorzugsweise metallisch. In oder an der Fluidleitung können auch zwei leitfähige Schichten oder leitfähige Litzen vorgesehen sein, um einen Stromkreis von der Steuervorrichtung durch die Fluidleitung zu dem Anschluss und zurück zu ermöglichen. Die leitfähige Litze oder die elektrisch leitfähige Schicht ist vorzugsweise wenigstens abschnittsweise mit einem elektrisch nicht leitfähigen Material beschichtet.

Es kann auch alternativ nur eine elektrische Leitung bzw. eine elektrische Verbindung vorgesehen sein und dann über einen Masseanschluss oder eine Masseleitung beispielsweise über das chirurgische Instrument ein entsprechender Strom abgeführt werden.

Vorzugsweise ist das Fluid eine Flüssigkeit.

Vorzugsweise weist der Fluidleitungsanschluss stirnseitig eine elektrisch leitende Kontaktfläche auf, die bei funktionsgemäßem Anschluss des Fluidleitungsanschlusses an das chirurgische Instrument in elektrischem Kontakt mit einer elektrisch leitenden Fläche des chirurgischen Instruments ist. Hierbei kann es vorzugsweise vorgesehen sein, dass zwei elektrisch leitende Kontaktflächen an der Stirnseite des Fluidleitungsanschlusses vorgesehen sind, die durch Kontakt mit dem chirurgischen Instrument elektrisch miteinander verbunden werden oder sind.

Vorzugsweise umfasst eine Aufbereitungsvorrichtung zur Aufbereitung von chirurgischen Instrumenten ein erfindungsgemäßes Überwachungssystem.

Die Aufgabe wird ferner durch ein Verfahren zum Verbinden eines chirurgischen Instruments mit wenigstens einer Fluidleitung, die beim Aufbereiten des chirurgischen Instruments Fluid zum chirurgischen Instrument führt oder vom chirurgischen Instrument wegführt, gelöst, wobei die wenigstens eine Fluidleitung an wenigstens einem Anschluss des chirurgischen Instrumentes angeschlossen wird, wobei mittels einer Steuervorrichtung überprüft wird, ob ein funktionsgemäßer Anschluss der wenigstens einen Fluidleitung mit dem wenigstens einen Anschluss des chirurgischen Instruments vorliegt, woraufhin die Steuervorrichtung ein Signal generiert, wobei ein Fluidleitungsanschluss der Fluidleitung bei funktionsgemäßem Anschluss an das chirurgische Instrument einen elektrischen Kontakt schließt, das dadurch weitergebildet ist, dass der elektrische Kontakt dadurch ermöglicht wird, dass eine Außenfläche der Fluidleitung, die elektrisch leitend ist, mit einer Außenfläche des chirurgischen Instruments oder eines Instrumentenanschlusses des chirurgischen Instruments, der wenigstens abschnittsweise leitend ist, einen Kontakt schließt.

Hierbei wird erfindungsgemäß festgestellt, ob ein funktionsgemäßer Anschluss der wenigstens einen Fluidleitung mit dem wenigstens einen Anschluss des chirurgischen Instruments vorliegt, beispielsweise durch Schalten eines Schalters, der in einer Endmontagestellung einer Fluidleitung nach dem Verbinden mit dem chirurgischen Instrument eingeschaltet wird, oder aufgrund eines Kontaktes von elektrischen Leitungen, so dass so auch bei funktionsgemäßem Anschluss der wenigstens einen Fluidleitung mit dem wenigstens einen Anschluss des chirurgischen Instruments ein elektrischer Kontakt geschlossen wird. Vorzugsweise umfasst die Steuervorrichtung eine Prüfvorrichtung oder ist eine Prüfvorrichtung.

Vorzugsweise wird bei einem nicht funktionsgemäßen Anschluss der Fluidleitung mit dem chirurgischen Instrument eine Fehlermeldung erzeugt. Beispielsweise kann die Fehlermeldung erzeugt werden, wenn nach einem Start des Anschließens bzw. des Anschließvorganges durch eine Bedienperson eine vorgebbare Zeit abgelaufen ist, ohne dass es zu einem funktionsgemäßen Anschluss der Fluidleitung gekommen ist. Es kann auch vorgesehen sein, dass die Steuervorrichtung ein Computerprogramm oder eine Warteschleife startet, sobald eine Bedienperson eingibt, dass diese nun ein chirurgisches Instrument mit einer Fluidleitung oder mit mehreren Fluidleitungen anschließen möchte. Das Programm kann hierbei die Möglichkeit vorsehen, der Bedienperson eine Anleitung zum ordnungsgemäßen Anschließen von Fluidleitungen oder einer Fluidleitung an das chirurgische Instrument vorzugeben. Die Bedienperson kann dann sukzessive die Fluidleitungen anschließen und nach jedem Anschließen einer Fluidleitung kann beispielsweise eine Prüftaste von der Bedienperson betätigt werden oder ein Initialisierungsbefehl eingegeben werden, um zu überprüfen, ob ein funktionsgemäßer Anschluss vorliegt.

Es kann auch vorgesehen sein, dass in dem Moment, in dem das chirurgische Instrument in den Bereich einer Bestückungsstation befördert wird, automatisch erkannt wird, um welches chirurgische Instrument es sich handelt, so dass für das Überwachungssystem eindeutig ist, welche Fluidanschlüsse anzuschließen sind. Dieses kann einer Bedienperson durch das Überwachungssystem zur Kenntnis gebracht werden, beispielsweise über die Anzeige auf einer Anzeigeeinheit. Hierzu kann das chirurgische Instrument mit Identifizierungsmerkmalen, wie beispielsweise einem RFID-Chip versehen sein, über die eine eindeutige Identifizierung des chirurgischen Instruments möglich ist. Diese Bestückungsstation kann eine Vorrichtung sein, die räumlich getrennt von einer Aufbereitungsvorrichtung ist, und dient beispielsweise dazu, Körbe zum Einbringen in eine Aufbereitungsvorrichtung bequem und sicher mit chirurgischen Instrumenten, die aufzubereiten sind, zu bestücken.

Vorzugsweise ist das Signal ein Bestätigungssignal, das von einer Steuervorrichtung, insbesondere einer Aufbereitungsvorrichtung, generiert wird, insbesondere wenn ein an einem Fluidleitungsanschluss vorgesehener elektrischer Kontakt geschlossen wird. Das Bestätigungssignal kann beispielsweise ein Ton sein.

Vorzugsweise wird zum Anschließen mehrerer Fluidleitungen an das chirurgische Instrument eine Darstellung der vorzunehmenden Anschlüsse auf einer Anzeigevorrichtung, insbesondere des Überwachungssystems, zur Verfügung gestellt, wobei nach einem Quittieren eines vorgenommenen Anschlusses durch die Bedienperson von der Steuervorrichtung überprüft wird, ob der vorgenommene Anschluss funktionsgemäß ist. Vorzugsweise wird für den Fall, dass der Anschluss nicht funktionsgemäß ist, eine dem vorgenommenen Anschluss zugeordnete Fehlermeldung generiert. Bei der Fehlermeldung kann es sich um eine optische Anzeige oder auch ein akustisches Signal handeln.

Vorzugsweise wird der fehlerhafte Anschluss auf der Anzeigevorrichtung angezeigt.

Zudem kann ein Verfahren zum Betreiben einer Aufbereitungsvorrichtung zum Aufbereiten eines chirurgischen Instruments mit einem vorgenannten Verfahren zum Verbinden eines chirurgischen Instruments mit wenigstens einer Fluidleitung ausgeführt werden, wobei nur bei funktionsgemäßem Anschluss aller Fluidleitungen eine Aufbereitung des chirurgischen Instruments begonnen wird. Bei einer Aufbereitung des chirurgischen Instruments wird insbesondere ein Fluid, vorzugsweise eine Flüssigkeit durch die Fluidleitung oder die Fluidleitungen gefördert.

Vorzugsweise wird überprüft, ob bei der Aufbereitung des chirurgischen Instruments bzw. beim Betrieb der Aufbereitungsvorrichtung der Anschluss oder die Anschlüsse der Fluidleitungen funktionsgemäß bleibt oder bleiben.

Durch die Erfindung ist es möglich, einen nicht korrekt angeschlossenen Adapter bzw. eine nicht korrekt angeschlossene Fluidleitung an ein chirurgisches Instrument unmittelbar beim Anschluss der Fluidleitung an das chirurgische Instrument zu erkennen. Hierzu werden an den Endstücken der Fluidleitung bzw. der Fluidleitungen oder den Fluidleitungsanschlüssen Kontakte, beispielsweise Stirnflächenkontakte, vorgesehen. Bei einem korrekt angeschlossenen Fluidleitungsanschluss schließt dann der vorgenommene Kontakt elektrisch. Der elektrisch geschlossene Kontakt wird beispielsweise durch die Steuervorrichtung durch eine Kombination aus akustischem und visuellem Feedback dargestellt.

Unmittelbar beim Anschließen des Fluidleitungsanschlusses bzw. der Fluidleitung an das chirurgische Instrument bekommt die Bedienperson durch die Steuervorrichtung ein akustisches Signal, beispielsweise einen einmaligen Ton bei einem korrekten Anschluss und einen wiederholten Ton bei einem fehlerhaften Anschluss. Hierbei ist es nicht notwendig, dass die Bedienperson den Blick von dem Fluidleitungsanschluss abwendet.

Zusätzlich macht es Sinn, auch ein visuelles Feedback beispielsweise auf einer Anzeigevorrichtung darzustellen, beispielsweise kann ein grüner Haken für jeden korrekt angeschlossenen Fluidleitungsanschluss bzw. jede korrekt angeschlossene Fluidleitung auf der Anzeigevorrichtung vorgesehen sein. Entsprechend kann für jede nicht korrekt angeschlossene Fluidleitung bzw. jeden nicht korrekt angeschlossenen Fluidleitungsanschluss ein rotes Kreuz dargestellt sein.

Die Bedienperson bekommt so vor dem eigentlichen Start eines Aufbereitungsprogramms eine Rückmeldung, ob das eingelegte chirurgische Instrument korrekt angeschlossen ist.

Vorzugsweise wird die Funktionalität des Überwachungssystems bzw. der Aufbereitungsvorrichtung mit einer automatischen Instrumentenerkennung erweitert. Hierbei erkennt das Überwachungssystem bzw. die Aufbereitungsvorrichtung das chirurgische Instrument und kann dadurch die korrekten Fluidleitungsanschlüsse auf eine Anzeigevorrichtung graphisch oder in Form einer Liste darstellen. An dieser Darstellung auf der Anzeigevorrichtung kann der erfolgreiche oder fehlerhafte Anschluss für jeden einzelnen Fluidleitungsanschluss bzw. für jede einzelne Fluidleitung visualisiert werden.

Die Erkennung, ob die Fluidleitung bzw. die Fluidleitungsanschlüsse korrekt angeschlossen sind, kann als vorgelagerter Programmschritt vor einem eigentlichen Aufbereitungsprozess vorgenommen werden. In diesem vorgelagerten Programmschritt werden beispielsweise die Stirnflächenkontakte der Fluidleitungen bzw. des Fluidleitungsanschlusses über die Steuervorrichtung aktiviert. Durch eine anschließende Deaktivierung der Kontakte kann ausgeschlossen werden, dass durch entsprechend eintretendes Wasser oder umlaufendes Wasser bei der Aufbereitung eine Fehldetektion auftritt.

Weitere Merkmale bevorzugter Ausführungsformen werden aus den beigefügten Zeichnungen ersichtlich. Bevorzugte Ausführungsformen werden nachstehend

Bezugnahme auf die Zeichnungen beschrieben, wobei bezüglich aller im Text nicht näher erläuterten Einzelheiten ausdrücklich auf die Zeichnungen verwiesen wird. Es zeigen:
- Fig. 1: eine schematische Darstellung eines Überwachungssystems,
- Fig. 2: eine schematische Darstellung eines Anschlusses einer Fluidleitung an ein chirurgisches Instrument in einer schematischen Ausschnittsdarstellung, und
- Fig. 3: eine schematische Schnittdarstellung durch eine schematische stirnseitige Ansicht einer erfindungsgemäßen Fluidleitung.

In den Zeichnungen sind jeweils gleiche oder gleichartige Elemente und/oder Teile mit denselben Bezugsziffern versehen, so dass von einer erneuten Vorstellung jeweils abgesehen wird.

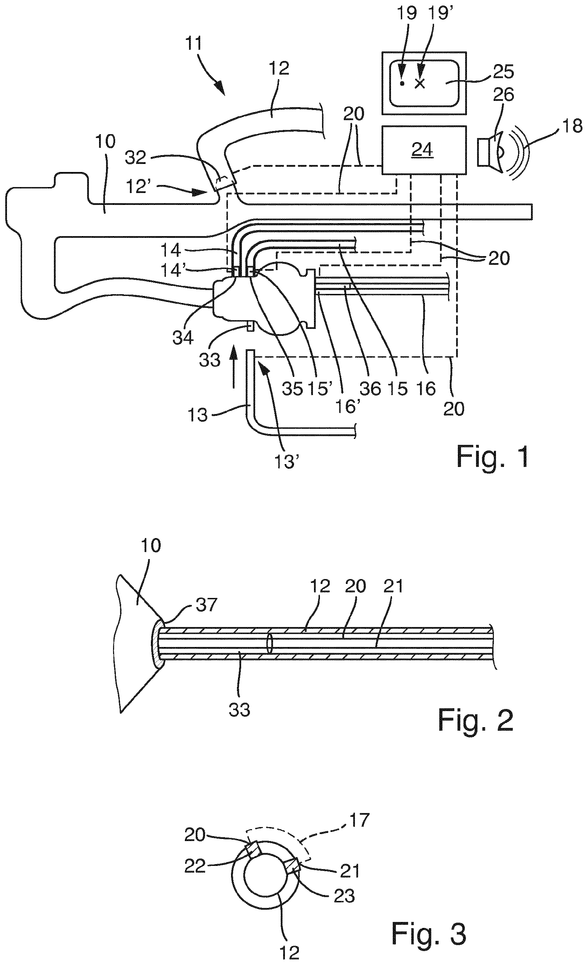

Fig. 1 zeigt schematisch ein Überwachungssystem 11 für den Anschluss eines chirurgischen Instruments 10 mit Fluidleitungen 12 - 16.

Chirurgische Instrumente, wie beispielsweise Endoskope, weisen Kanäle oder Lumen auf, die nach Benutzung des chirurgischen Instruments zu reinigen und zu desinfizieren sind. Hierzu gelangen diese Instrumente typischerweise in eine Aufbereitungsvorrichtung. In der Aufbereitungsvorrichtung wird von außen ein Reinigungsfluid auf das chirurgische Instrument geführt und zudem die entsprechenden Kanäle bzw. Lumen 23 unter anderem mit einer Reinigungsflüssigkeit gespült.

Um eine ordnungsgemäße Reinigung, d.h. eine komplette Reinigung und Desinfizierung, d.h. Aufbereitung des chirurgischen Instruments vornehmen zu können, ist es notwendig, dass die Fluidleitungen 12 - 16 mit deren stirnseitigen bzw. endseitigen Fluidleitungsanschlüssen 12' - 16' mit den Instrumentenanschlüssen 32 - 36 verbunden werden. Die Verbindung kann leckagefrei sein. Alternativ kann eine Verbindung mit definierter Leckage vorgesehen sein.

Um effizient eine Aufbereitung vornehmen zu können, ist erfindungsgemäß vorgesehen, beim Bestücken des chirurgischen Instruments bzw. der Instrumentenanschlüsse 32 - 36 mit den Fluidleitungsanschlüssen 12' - 16' bzw. mit der Fluidleitung 12 - 16 zu überwachen, ob die Anschlüsse funktionsgemäß sind, also insbesondere so, dass diese vollständig korrekt ausgeführt worden sind. Hierzu wird bei einem funktionsgemäßen Anschluss ein elektrischer Kontakt geschlossen. Der elektrische Kontakt kann beispielsweise dadurch geschlossen werden, dass, wie in Fig. 2 dargestellt ist, zwei elektrische Leitungen 20, 21, die auf dem Mantel der Fluidleitung 12 angeordnet sind, sich, wie in Fig. 3 dargestellt ist, bis zur Stirnseite erstecken und dort jeweils eine Endkontaktfläche 22 bzw. 23 bilden, die in elektrischen Kontakt mit einer elektrisch leitenden Fläche 37 des chirurgischen Instruments 10 gelangen. Hierdurch kann ein elektrischer Strom von einer Steuervorrichtung 24 über den geschlossenen elektrischen Kontakt zwischen den Endkontaktflächen 22 und 23 sowie der elektrisch leitenden Fläche 37 des Endoskops 10 geführt werden, wodurch festgestellt werden kann, dass ein entsprechender funktionsgemäßer Anschluss vorliegt. Alternativ kann ein Widerstand zwischen den elektrischen Leitungen 20 und 21 gemessen werden, wobei bei Unterschreiten eines vorgebbaren Wertes für den Widerstand von einem funktionsgemäßen Anschluss ausgegangen wird.

Diese Art des elektrischen Kontaktes ist nur eine Art von vielen denkbaren Möglichkeiten. Alternativ kann auch ein Schalter vorgesehen sein, der in der Fluidleitung oder an der Fluidleitung angeordnet ist und bei Kontakt mit einer Endfläche des chirurgischen Instruments geschlossen wird. Es kann alternativ auch vorgesehen sein, die Fluidleitung mit einem Fluidleitungsanschluss zu versehen, die in Art eines Bajonettverschlusses mit einem Instrumentenanschluss des chirurgischen Instruments 10 verbunden werden kann und nur bei entsprechendem vollständigen Verdrehen des Bajonetts in dem Gegenstück hierzu einen elektrischen Kontakt schließt.

In Fig. 1 ist ganz schematisch und allgemein gezeigt, wie entsprechende elektrische Leitungen 20 von dem Anschluss zwischen der Fluidleitung und dem chirurgischen Instrument zu einer Steuervorrichtung 24 geführt werden. Zudem ist beispielsweise die Fluidleitung 13 noch entfernt von dem Instrumentenanschluss 33. Die Fluidleitung 13 soll noch in Pfeilrichtung auf den Instrumentenanschluss 33 geschoben werden. Die entsprechenden Fluidleitungen 12 - 16 sind in Fig. 1 und 2 nur abschnittsweise dargestellt.

Die Steuervorrichtung kann bei entsprechendem funktionsgemäßen Anschluss des jeweiligen Fluidleitungsanschlusses an das chirurgische Instrument bzw. dem jeweiligen Instrumentenanschluss 32 - 36 über einen Lautsprecher 26 ein akustisches Signal 18 generieren und/oder auf der Anzeigevorrichtung 25 ein visuelles Signal 19 oder 19' generieren, die einer Bedienperson anzeigen, ob der Anschluss funktionsgemäß ist oder nicht.

Bei der Fluidleitung 12 - 16 kann es sich um Schläuche handeln, insbesondere kann es sich hierbei um flexible Fluidleitungen handeln.

Es ist in Fig. 3 schematisch gezeigt, wie ein elektrischer Kontakt 17 erzeugt wird. Dieser elektrische Kontakt 17 entsteht über die Endkontaktflächen 22 und 23, wenn diese an eine elektrisch leitende Fläche anstoßen. In diesem Fall wird ein elektrischer Kontakt 17 geschlossen, was über die entsprechenden mit den Endkontaktflächen 22 und 23 verbundenen elektrischen Leitungen 20 bzw. 21 der Steuervorrichtung 24 übermittelt wird.

Merkmale, die mit "insbesondere" oder "vorzugsweise" gekennzeichnet sind, sind als fakultative Merkmale zu verstehen.

### Bezugszeichenliste

- 10: chirurgisches Instrument
- 11: Überwachungssystem
- 12, 13, 14, 15, 16: Fluidleitung
- 12', 13', 14', 15', 16': Fluidleitungsanschluss
- 17: elektrischer Kontakt
- 18: akustisches Signal
- 19, 19': visuelles Signal
- 20, 21: elektrische Leitung
- 22, 23: Endkontaktfläche
- 24: Steuervorrichtung
- 25: Monitor
- 26: Lautsprecher
- 32, 33, 34, 35, 36: Instrumentenanschluss
- 37: elektrisch leitende Fläche

## Patentansprüche

1. Überwachungssystem (11) für die Aufbereitung von chirurgischen Instrumenten (10), wobei zur Zufuhr eines Fluids zu dem chirurgischen Instrument (10) eine Fluidleitung (12, 13, 14, 15, 16) vorgesehen ist, die mit dem chirurgischen Instrument (10) verbindbar oder verbunden ist, wobei die Fluidleitung (12 - 16) zum Verbinden mit dem chirurgischen Instrument (10) einen Fluidleitungsanschluss (12' - 16') aufweist, wobei der Fluidleitungsanschluss (12' - 16') bei funktionsgemäßem Anschluss an das chirurgische Instrument (10) einen elektrischen Kontakt (17) schließt, **dadurch gekennzeichnet, dass** der elektrische Kontakt (17) dadurch ermöglicht wird, dass eine Außenfläche der Fluidleitung (12 - 16), die elektrisch leitend ist, mit einer Außenfläche des chirurgischen Instruments (10) oder eines Instrumentenanschlusses (32 - 36) des chirurgischen Instruments (10), der wenigstens abschnittsweise leitend ist, einen Kontakt schließt.

2. Überwachungssystem (11) nach Anspruch 1, **dadurch gekennzeichnet, dass** beim Schließen des elektrischen Kontakts (17) ein von einer Bedienperson wahrnehmbares Signal (18, 19) generiert wird.

3. Überwachungssystem (11) nach Anspruch 1 oder 2, **dadurch gekennzeichnet, dass** eine elektrische Verbindung (20, 21) von dem elektrischen Kontakt (17), insbesondere über die Fluidleitung (12 - 16), zu einer Steuervorrichtung (24) des Überwachungssystems (11) vorgesehen ist.

4. Überwachungssystem (11) nach einem der Ansprüche 1 bis 3, **dadurch gekennzeichnet, dass** die Fluidleitung (12 - 16) ein Schlauch ist.

5. Überwachungssystem (11) nach einem der Ansprüche 1 bis 4, **dadurch gekennzeichnet, dass** die elektrische Verbindung (20, 21) eine in oder an der Fluidleitung (12 - 16) angeordnete leitfähige Litze oder eine leitfähige Schicht ist oder umfasst.

6. Überwachungssystem (11) nach einem der Ansprüche 1 bis 5, **dadurch gekennzeichnet, dass** der Fluidleitungsanschluss (12' - 16') stirnseitig eine elektrisch leitende Kontaktfläche (22, 23) aufweist, die bei funktionsgemäßem Anschluss des Fluidleitungsanschlusses (12' - 16') an das chirurgische Instrument (10) in elektrischem Kontakt mit einer elektrisch leitenden Fläche (37) des chirurgischen Instruments (10) ist.

7. Aufbereitungsvorrichtung zur Aufbereitung von chirurgischen Instrumenten (10) umfassend ein Überwachungssystem (11) nach einem der Ansprüche 1 bis 6.

8. Verfahren zum Verbinden eines chirurgischen Instruments (10) mit wenigstens einer Fluidleitung (12 - 16), die beim Aufbereiten des chirurgischen Instruments (10) Fluid zum chirurgischen Instrument (10) führt oder vom chirurgischen Instrument (10) wegführt, wobei die wenigstens eine Fluidleitung (12 - 16) an wenigstens einem Anschluss (32 - 36) des chirurgischen Instrumentes (10) angeschlossen wird, wobei mittels einer Steuervorrichtung (24) überprüft wird, ob ein funktionsgemäßer Anschluss der wenigstens einen Fluidleitung (12 - 16) mit dem wenigstens einen Anschluss (32 - 36) des chirurgischen Instruments (10) vorliegt, woraufhin die Steuervorrichtung (24) ein Signal (18, 19') generiert, wobei ein Fluidleitungsanschluss (12' - 16') der Fluidleitung (12 - 16) bei funktionsgemäßem Anschluss an das chirurgische Instrument (10) einen elektrischen Kontakt (17) schließt, **dadurch gekennzeichnet, dass** der elektrische Kontakt (17) dadurch ermöglicht wird, dass eine Außenfläche der Fluidleitung (12 - 16), die elektrisch leitend ist, mit einer Außenfläche des chirurgischen Instruments (10) oder eines Instrumentenanschlusses (32 - 36) des chirurgischen Instruments (10), der wenigstens abschnittsweise leitend ist, einen Kontakt schließt.

9. Verfahren nach Anspruch 8, **dadurch gekennzeichnet, dass** bei einem nicht funktionsgemäßen Anschluss der Fluidleitung (12 - 16) mit dem chirurgischen Instrument (10) eine Fehlermeldung (19') erzeugt wird.

10. Verfahren nach Anspruch 8, **dadurch gekennzeichnet, dass** das Signal (18, 19, 19') ein Bestätigungssignal (18, 19) ist, das von einer Steuervorrichtung (24), insbesondere einer Aufbereitungsvorrichtung, generiert wird, insbesondere wenn ein an einem Fluidleitungsanschluss (12' - 16') vorgesehener elektrischer Kontakt (17) geschlossen wird.

11. Verfahren nach einem der Ansprüche 8 bis 10, **dadurch gekennzeichnet, dass** zum Anschließen mehrerer Fluidleitungen (12 - 16) an das chirurgische Instrument (10) eine Darstellung der vorzunehmenden Anschlüsse auf einer Anzeigevorrichtung (25) zur Verfügung gestellt wird, wobei nach einem Quittieren eines vorgenommenen Anschlusses durch eine Bedienperson von der Steuervorrichtung (24) überprüft wird, ob der vorgenommene Anschluss funktionsgemäß ist.

12. Verfahren nach Anspruch 11, **dadurch gekennzeichnet, dass** für den Fall, dass der Anschluss nicht funktionsgemäß ist, eine dem vorgenommenen Anschluss zugeordnete Fehlermeldung (19') generiert wird.

13. Verfahren nach Anspruch 12, **dadurch gekennzeichnet, dass** der fehlerhafte Anschluss auf der Anzeigevorrichtung (25) angezeigt wird.

14. Verfahren zum Betreiben einer Aufbereitungsvorrichtung zum Aufbereiten eines chirurgischen Instruments (10), wobei ein Verfahren nach einem der Ansprüche 8 - 12 ausgeführt wird und wobei nur bei funktionsgemäßem Anschluss aller Fluidleitungen (12 - 16) eine Aufbereitung des chirurgischen Instruments (10) begonnen wird.

15. Verfahren nach Anspruch 14, **dadurch gekennzeichnet, dass** bei der Aufbereitung des chirurgischen Instruments (10) überprüft wird, ob der Anschluss der Fluidleitungen (12 - 16) während der Aufbereitung funktionsgemäß bleibt.

## Claims

1. A monitoring system (11) for reprocessing surgical instruments (10), wherein a fluid line (12, 13, 14, 15, 16) is provided for supplying a fluid to the surgical instrument (10), which fluid line can be or is connected to the surgical instrument (10), wherein the fluid line (12 - 16) has a fluid line connection (12' - 16') for connecting to the surgical instrument (10), wherein the fluid line connection (12' - 16') closes an electrical contact (17) when it is functionally connected to the surgical instrument (10), **characterized in that** the electrical contact (17) is made possible **in that** an external face of the fluid line (12 - 16), which is electrically conductive, closes a contact with an external face of the surgical instrument (10) or of an instrument connection (32 - 36) of the surgical instrument (10) which is at least partially conductive.

2. The monitoring system (11) according to Claim 1, **characterized in that** when closing the electrical contact (17) a signal (18, 19) which can be detected by an operator is generated.

3. The monitoring system (11) according to Claim 1 or 2, **characterized in that** an electrical connection (20, 21) is provided from the electrical contact (17), in particular via the fluid line (12 - 16), to a control device (24) of the monitoring system (11).

4. The monitoring system (11) according to any one of Claims 1 to 3, **characterized in that** the fluid line (12 - 16) is a hose.

5. The monitoring system (11) according to any one of Claims 1 to 4, **characterized in that** the electrical connection (20, 21) is or comprises a conductive strand arranged in or on the fluid line (12 - 16) or a conductive layer.

6. The monitoring system (11) according to any one of Claims 1 to 5, **characterized in that** the fluid line connection (12' - 16') has an electrically conductive contact surface (22, 23) on the front face, which contact surface in the case of a functional connection of the fluid line connection (12' - 16') to the surgical instrument (10) is in electrical contact with an electrically conductive surface (37) of the surgical instrument (10).

7. A reprocessing device for reprocessing surgical instruments (10) comprising a monitoring system (11) according to any one of Claims 1 to 6.

8. A method for connecting a surgical instrument (10) to at least one fluid line (12 - 16) which, when the surgical instrument (10) is reprocessed, conducts fluid to the surgical instrument (10) or conducts fluid away from the surgical instrument (10), wherein the at least one fluid line (12 - 16) is connected to at least one connection (32 - 36) of the surgical instrument (10), wherein it is verified by means of a control device (24) whether a functional connection of the at least one fluid line (12 - 16) to the at least one connection (32 - 36) of the surgical instrument (10) exists, whereupon the control device (24) generates a signal (18, 19'), wherein a fluid line connection (12' - 16') of the fluid line (12 - 16) closes an electrical contact (17) when it is functionally connected to the surgical instrument (10), **characterized in that** the electrical contact (17) is made possible **in that** an external surface of the fluid line (12 - 16), which is electrically conductive, closes a contact with an external face of the surgical instrument (10) or of an instrument connection (32 - 36) of the surgical instrument (10) which is at least partially conductive.

9. The method according to Claim 8, **characterized in that** an error message (19') is produced in the case of a non-functional connection of the fluid line (12 - 16) to the surgical instrument (10).

10. The method according to Claim 8, **characterized in that** the signal (18, 19, 19') is a confirmation signal (18, 19) which is generated by a control device (24), in particular a reprocessing device, in particular when an electrical contact (17) provided on a fluid line connection (12' - 16') is closed.

11. The method according to any one of Claims 8 to 10, **characterized in that** for connecting multiple fluid lines (12 - 16) to the surgical instrument (10) a representation of the connections to be made is provided on a display device (25), wherein after an operator has acknowledged a connection which has been made it is verified by the control device (24) whether the connection which has been made is functional.

12. The method according to Claim 11, **characterized in that** in the event that the connection is not functional, an error message (19') which is assigned to the connection which has been made is generated.

13. The method according to Claim 12, **characterized in that** the faulty connection is displayed on the display device (25).

14. A method for operating a reprocessing device for reprocessing a surgical instrument (10), wherein a method according to any one of Claims 8 - 12 is executed and wherein a reprocessing of the surgical instrument (10) is only initiated in the case of a functional connection of all fluid lines (12 - 16).

15. The method according to Claim 14, **characterized in that** when the surgical instrument (10) is reprocessed, it is verified whether the connection of the fluid lines (12 - 16) remains functional during the reprocessing.

## Revendications

1. Système de surveillance (11) pour le traitement d'instruments chirurgicaux (10), dans lequel, pour l'amenée d'un fluide à l'instrument chirurgical (10), il est prévu une conduite de fluide (12, 13, 14, 15, 16) qui est apte à être reliée ou qui est reliée à l'instrument chirurgical (10), la conduite de fluide (12 - 16) présentant un raccord (12' - 16') de conduite de fluide pour le raccordement à l'instrument chirurgical (10), le raccord (12' - 16') de conduite de fluide fermant un contact électrique (17) lors d'un raccordement fonctionnel à l'instrument chirurgical (10), **caractérisé en ce que** le contact électrique (17) est rendu possible par le fait qu'une surface extérieure de la conduite de fluide (12 - 16), qui est électriquement conductrice, ferme un contact avec une surface extérieure de l'instrument chirurgical (10) ou un raccord d'instrument (32 - 36) de l'instrument chirurgical (10), qui est conducteur au moins partiellement.

2. Système de surveillance (11) selon la revendication 1, **caractérisé en ce que** lors de la fermeture du contact électrique (17), un signal (18, 19) perceptible par un opérateur est généré.

3. Système de surveillance (11) selon la revendication 1 ou la revendication 2, **caractérisé en ce qu'**une liaison électrique (20, 21) est prévue entre le contact électrique (17), notamment via la conduite de fluide (12 - 16), et un dispositif de commande (24) du système de surveillance (11).

4. Système de surveillance (11) selon l'une des revendications 1 à 3, **caractérisé en ce que** la conduite de fluide (12 - 16) est un tuyau flexible.

5. Système de surveillance (11) selon l'une des revendications 1 à 4, **caractérisé en ce que** la connexion électrique (20, 21) est, ou comprend, un toron conducteur ou une couche conductrice agencé(e) dans ou sur la conduite de fluide (12 - 16).

6. Système de surveillance (11) selon l'une des revendications 1 à 5, **caractérisé en ce que** le raccord (12' - 16') de conduite de fluide présente, côté frontal, une surface de contact (22, 23) électriquement conductrice, qui, lorsque le raccord (12' - 16') de conduite de fluide est raccordé de manière fonctionnelle à l'instrument chirurgical (10), est en contact électrique avec une surface électriquement conductrice (37) de l'instrument chirurgical (10).

7. Dispositif de traitement pour le traitement d'instruments chirurgicaux (10) comprenant un système de surveillance (11) selon l'une des revendications 1 à 6.

8. Procédé de raccordement d'un instrument chirurgical (10) à au moins une conduite de fluide (12 - 16) qui, lors du traitement de l'instrument chirurgical (10), amène du fluide à l'instrument chirurgical (10) ou l'éloigne de l'instrument chirurgical (10), ladite au moins une conduite de fluide (12 - 16) étant raccordée à au moins un raccord (32 - 36) de l'instrument chirurgical (10), un dispositif de commande (24) permettant de vérifier si ladite au moins une conduite de fluide (12 - 16) est raccordée de manière fonctionnelle à ledit au moins un raccord (32 - 36) de l'instrument chirurgical (10), après quoi le dispositif de commande (24) génère un signal (18, 19'), un raccord de conduite de fluide (12' - 16') de la conduite de fluide (12 - 16) fermant un contact électrique (17) en cas de raccordement fonctionnel à l'instrument chirurgical (10), **caractérisé en ce que** le contact électrique (17) est rendu possible de telle sorte qu'une surface extérieure de la conduite de fluide (12 - 16), qui est électriquement conductrice, ferme un contact avec une surface extérieure de l'instrument chirurgical (10) ou d'un raccord d'instrument (32 - 36) de l'instrument chirurgical (10), qui est conducteur au moins partiellement.

9. Procédé selon la revendication 8, **caractérisé en ce qu'**un message d'erreur (19') est généré en cas de raccordement non fonctionnel de la conduite de fluide (12 - 16) à l'instrument chirurgical (10).

10. Procédé selon la revendication 8, **caractérisé en ce que** le signal (18, 19, 19') est un signal de confirmation (18, 19) généré par un dispositif de commande (24), notamment un dispositif de traitement, notamment lorsqu'un contact électrique (17) prévu sur un raccord (12' - 16') de conduite de fluide est fermé.

11. Procédé selon l'une des revendications 8 à 10, **caractérisé en ce que**, pour raccorder plusieurs conduites de fluide (12 - 16) à l'instrument chirurgical (10), une représentation des raccordements à effectuer est prévue sur un dispositif d'affichage (25), le dispositif de commande (24) vérifiant, après une confirmation d'un raccordement effectué par un opérateur, si le raccordement effectué est conforme au fonctionnement.

12. Procédé selon la revendication 11, **caractérisé en ce que**, dans le cas où le raccordement n'est pas conforme au fonctionnement, un message d'erreur (19') associé au raccordement effectué est généré.

13. Procédé selon la revendication 12, **caractérisé en ce que** le raccordement défectueux est affiché sur le dispositif d'affichage (25).

14. Procédé d'exploitation d'un dispositif de traitement pour le traitement d'un instrument chirurgical (10), dans lequel un procédé selon l'une des revendications 8 à 12 est mis en oeuvre, et dans lequel on ne commence un traitement de l'instrument chirurgical (10) que si toutes les conduites de fluide (12 à 16) sont raccordées de manière conforme au fonctionnement.

15. Procédé selon la revendication 14, **caractérisé en ce que**, lors du traitement de l'instrument chirurgical (10), on vérifie si le raccordement des conduites de fluide (12 - 16) reste conforme au fonctionnement pendant le traitement.
